# EUROPEAN PATENT APPLICATION

(11) **EP 2 907 875 A1**
(43) Date of publication of application: **19.08.2015**
(21) Application number: 13075089.6
(22) Date of filing: 15.11.2013
(51) Int. Cl.: C12N 15/63, C12Q 1/68, C12N 15/85, C12N 15/90

(54) **A novel method to load a mammalian artificial chromosome with multiple genes**

(30) Priority: 16.11.2012 HU P1200662
(71) Applicant: Magyar Tudományos Akadémia Szegedi Biológiai Kutatoközpont 33%, 6726 Szeged (HU)
(72) Inventor: Katona, Róbert, 6754 Újszentiván (HU); Hadlaczky, Gyula, 6723 Szeged (HU); Fodor, Katalin, 6723 Szeged (HU); Praznovszky, Tünde, 6723 Szeged (HU); Blazsó, Péter, 6722 Szeged (HU); Udvardy, Andor, 6726 Szeged (HU); Mózesné Holló, Gyöngyi, 6726 Szeged (HU); Keresó, Judit, 6725 Szeged (HU)
(74) Representative: Svingor, Adam

(57) **Abstract**

The invention provides for a system for uploading genes of interest into an artificial chromosome expression system (ACE) that has been engineered to include multiple sites for site-specific, recombination directed integration, whereby a second or further gene of interest can be loaded onto the ACE by a targeting vector, through the acceptor recombination site(s) on said ACE.

## Description

### FIELD OF THE INVENTION

Methods for loading genes of interest into an artificial chromosome expression system (ACE) that has been engineered to include multiple sites for site-specific, recombination directed integration, whereby a second or further gene of interest can be loaded onto the ACE by a targeting vector, through the acceptor recombination site(s) on said ACE, whereas selectable markers applied can be removed from and each integrated genes of interest can be maintained on said ACE are provided. Thus, the novel method according to the invention makes it possible to load a practically unlimited number of genes onto ACEs by using only two selectable marker genes that could be removed from the ACEs. The invention is useful for: *i*, gene therapeutic applications aimed at the treatment of simple and complex disorders and cancers *ii,* cell therapy applications including: stem cell research, iPSC (induced pluripotent stem cell) production and research, production of differentiated cell lines from pluripotent and multipotent stem cells to study the differentiation process and also produce differentiated cell types for cell therapy *iii,* multiple protein productions from one producer cell line for basic research and industry iv, production of immortalized and transiently immortalized somatic cell lines which are difficult to expand by differentiation methods or direct ACE delivery v, single and multiple gene overexpression research vi, single and multiple gene knock-down research.

### BACKGROUND ART

Mammalian artificial chromosomes (MACs) are natural chromosome-based vectors that can replicate in mammalian cells just as endogenous chromosomes and are capable to carry vast amount of recombinant genetic material. They are reasonably stable and typically segregate well in both mitosis and meiosis. MACs typically comprise a mammalian centromere, a region of specialized chromatin found within the chromosome that provides the foundation for the assembly of kinetochore, a protein structure where upon mitosis or meiosis the spindle fibers attach before segregation of sister chromatids. Thus, a centromere confers the ability to a chromosome to segregate to daughter cells.

In higher eukaryotic cells, in particular in animal and plant cells, the boundary of a centromere is formed by highly repetitive DNA sequences which, via DNA binding proteins, create a genetically inactive zone around the centromere called pericentric heterochromatin. While no specific gene expression is observed in this region, the centromere could be detected by specific antibodies. Hadlaczky et al. [Proc.Natl.Acad.Sci.U.S.A 88, 8106-8110 (1991)] in 1991 described the isolation of a fragment of putative centromeric DNA by using such anti-centromere antibodies. By co-transfecting this DNA with a selectable marker gene into a cell line, dicentric chromosomes, carrying an additional functional centromere, were obtained. The presence of two functional centromeres in the same chromosome caused specific breakages that resulted in the regular appearance of a minichromosome. Praznovszky et al. [De novo chromosome formation in rodent cells. Proc. Natl. Acad. Sci. U.S.A 88, 11042-11046 (1991)] observed that incorporation of a heterologous DNA fragment resulted in the generation of an excess centromere. Thus, they could achieve *de novo* chromosome formation in cells in which the marker centromere was separated from the dicentric chromosome and formed a stable, full-sized functional chromosome by the so-called amplification process. In EP0473253 Hadlaczky et al. teach a non-human mammalian cell line that carries a chromosome containing an excess centromere comprising a human DNA sequence associated with a dominant selectable marker gene. As mentioned above, regions of heterochromatin surrounding the centromere comprise highly repetitive satellite DNAs, which are transcriptionally inactive. This feature was utilized in WO1997040183, wherein Hadlaczky G. and Szalay A. disclosed satellite DNA based artificial chromosomes (SATACs) useful as gene expression vectors. SATACs are typically made up predominantly of repeating units of short satellite DNA, however, do not contain protein coding gene sequences unless heterologous or foreign DNA is inserted into them. These artificial chromosomes comprising foreign DNA, e.g. a selectable marker gene provided a promising platform to develop expression vectors. The potential use of SATACs in gene therapy at that time was reviewed by Hadlaczky G [Hadlaczky, G. Satellite DNA-based artificial chromosomes for use in gene therapy. Curr.Opin.Mol.Ther 3, 125-132 (2001)].

Related patents, including issued U.S. Patent Nos. 6,077,697 and 6,025,155 (granted based on, among others, US patent applications 08/695,191 and 08/835,682, describe the generation of *ACes* (also designated satellite artificial chromosomes (SATACs)) from any species.

In US2012064578 published patent application Lindenbaum et al. described an artificial chromosome expression system (ACEs) called platform ACEs developed from SATACs, which comprised multiple site-specific recombination sites. Thereby it became possible to introduce multiple transgenes into an artificial chromosome provided that specific and different selection marker gene was applied for each transgene. The recombination site used by Lindenbaum et al. was the lambda att site designed for recombination directed integration in the presence of lambda integrase. This recombination site is unidirectional i.e. facilitates integration, while effectively prevents the excision of already integrated sequences upon further exposure to the integrase [see also Lindenbaum M. et al. "A mammalian artificial chromosome engineering system (ACE System) applicable to biopharmaceutical protein production, transgenesis and gene-based cell therapy" Nucleic Acids Research, 2004, Vol. 32, No. 21]. Kuroiwa Y et al. [Manipulation of human minichromosomes to carry greater than megabase-sized chromosome inserts. Nature Biotechnol (2000) 18:1086-1090] described the use of a bidirectional recombination system, the cre/lox homologous recombination system for the engineering of a human minichromosome. However, this site was later found disadvantageous in this field, on the one hand because of the possibility of excision and on the other hand due to a lower recombination activity [Yamaguchi S et al. "A Method for Producing Transgenic Cells Using a Multi-Integrase System on a Human Artificial Chromosome Vector" PLoS ONE 6(2) 17267 (2011)].

In summary, significant efforts have been made in the art to create mammalian artificial chromosomes a safe, stable and reliable genetic vector [Csonka, E. et al. Novel generation of human satellite DNA-based artificial chromosomes in mammalian cells. J.Cell Sci 113 (Pt 18), 3207-3216 (2000); Hadlaczky, G Curr.Opin.Mol.Ther 3, 125-132 (2001); Lindenbaum, M. et al. Nucleic Acids Res 32, e172 (2004); Duncan, A. & Hadlaczky, G. Chromosomal engineering. Curr. Opin. Bioteclmol 18, 420-424 (2007)]. In a most recent paper Kennard M. L. reviews the future prospects of use of engineered mammalian chromosomes in cellular protein production and finds that to date "the most successful technique has been based on the artificial chromosome expression or ACE System, which consists of the targeted transfection of cells containing mammalian based artificial chromosomes with multiple recombination acceptor sites" and this system eliminates the need for random integration into native host chromosomes [Kennard M. L. Mammalian Chromosome Engineering; Methods in Molecular Biology 738 (2011) 217-238]. These vectors, however, still prove to be imperfect if multiple transgenes are to be introduced as each of them requires the application of specific foreign sequences required for loading, like selectable marker genes, remaining on the loaded artificial chromosome.

Gene therapy might be an extremely promising field for applications of such vectors. In fact, recently a novel gene therapy method was developed called combined mammalian artificial chromosome-stem cell therapy and it has been shown that the ACE system is suitable for the treatment of an animal model for a devastating human disorder, Krabbe's disease, by delivering a therapeutic gene into mutant mice. Treated mutant mice lived more than four times longer. This gene therapy method used a novel therapeutic approach called combined mammalian artificial chromosome-stem cell therapy (CoMAC-Stem) [Katona, R.L. et al. A combined artificial chromosome-stem cell therapy method in a model experiment aimed at the treatment of Krabbe's disease in the Twitcher mouse. Cell. Mol. Life Sci 65, 3830-3838 (2008)]. The art still needs, however, an efficient vector system, e.g. for gene therapy applications, wherein multiple transgenes, e.g therapeutic genes are required.

The present inventors have recognized that it is possible to load multiple, in principle unlimited number of genes onto any chromosome vectors engineered with appropriate recombination/acceptor sites. In particular genes are loaded onto artificial chromosome expression systems (ACEs) by using a restricted number of, preferably only two selectable marker genes that could be removed from the ACEs. It has been also recognized that this is particulary desired before therapeutic applications. The present inventors have also found that only a small number of, preferably only two selectable marker genes are sufficient to load multiple of genes onto said artificial chromosomes if a given arrangement of elements in a loading vector as disclosed herein (the superloading vector) is applied and these selectable marker genes then can be removed from the artificial chromosomes.

### BRIEF DESCRIPTION OF THE INVENTION

Presently for each therapeutic gene, which is being loaded onto the ACEs, a new selection marker gene should be present. Complex diseases require the cooperative action of several genes for treatment. There is a limited number of selection marker genes available and also there is a risk of serious side effects caused by the unwanted expression of these marker genes in mammalian cells, organs and organisms. We show here a novel method that makes it possible to load unlimited number of genes onto ACEs by using only two selectable marker genes that could be removed from the ACEs, before therapeutic applications. This novel technology could revolutionize gene therapeutic applications aimed at the treatment of complex disorders and cancers. It could also speed up cell therapy by allowing researchers to engineer a chromosome with a predetermined set of genetic factors that could help differentiate adult stem cells, embryonic stem cells and iPS cells into adult cell types with therapeutic value.

Thus, the invention relates to a system for loading genes of interest into an artificial chromosome expression system (ACE) that has been engineered to include multiple sites for site-specific recombination directed integration
said system comprising at least
i) an ACE having
   - multiple (preferably 2 to 200, more preferably 50 to 200) recombination acceptor sites and
   - a promoter directly (preferably at most 50 bp, 45 bp, 43 bp, 40 bp, 35 bp or 30 bp) upstream from the recombination acceptor sites, and
ii) a targeting expression vector (superloading vector), said targeting vector having
   - a recombination donor site,
   - a transgene expression cassette for the expression of a gene of interest comprising at least one gene of interest or at least one cloning site for a gene of interest operably linked with a promoter-enhancer element allowing expression of the gene(s) of interest
   - a positive selectable marker gene directly downstream from the recombination donor site so that said positive selectable marker gene is expressed under the control of the promoter upstream from the recombination acceptor site of the ACE in a mammalian cell, once the targeting vector has been integrated into the ACE to form a loaded ACE
   - a negative selectable marker gene expression cassette including promoter sequence and poly-A signal to ensure the expression of the negative selectable marker gene,
      wherein the loaded ACE comprises a removable DNA segment, said removable DNA segment being
   - flanked by an upstream and a downstream deletion recombination site for removal of said DNA segment from the loaded ACE upon the enzymatic action of a deleting recombinase enzyme specific to (capable to fulfill of its enzymatic reaction on) the deleting recombination sites thereby removing the removable DNA segment, and
   - comprising both selectable marker genes.

The invention also relates to targeting vector for loading genes of interest into an artificial chromosome expression system (ACE) that has been engineered to include multiple sites for site-specific, recombination directed integration, said ACE having
- multiple (preferably 2 to 200, more preferably 50 to 200) recombination acceptor sites and
- a promoter directly (preferably at most 50 bp, 45 bp, 43 bp, 40 bp, 35 bp or 30 bp) upstream from the recombination acceptor sites,
   and said targeting vector (superloading vector) having
- a recombination donor site,
- a transgene expression cassette for the expression of a gene of interest comprising at least one gene of interest or at least one cloning site for a gene of interest operably linked with a promoter-enhancer element allowing expression of the gene(s) of interest
- a positive selectable marker gene directly downstream from the recombination donor site so that said positive selectable marker gene is expressed under the control of the promoter upstream from the recombination acceptor site of the ACE in a mammalian cell, once the targeting vector has been integrated into the ACE to form a loaded ACE
- a negative selectable marker gene expression cassette including promoter sequence and poly-A signal to ensure the expression of the negative selectable marker gene,
   wherein the loaded ACE comprises a removable DNA segment, said removable DNA segment being
- flanked by an upstream and a downstream deletion recombination site for removal of said DNA segment from the loaded ACE upon the enzymatic action of a deleting recombinase enzyme specific to (capable to fulfill of its enzymatic reaction on) the deleting recombination sites thereby removing the removable DNA segment, and
- comprising both selectable marker genes.

Preferably, in the system or in the targeting vector according to the invention both the upstream and downstream deletion recombination sites are LoxP recombination sites in direct orientation for the enzymatic action of Cre recombinase enzyme, which enzyme, if required, is capable to fulfill of its enzymatic reaction on the LoxP sites and by this step both the positive selectable marker gene and the negative selectable marker gene cassette can be removed from the ACE chromosome.

Preferably, in the targeting vector according to the invention
- the downstream deletion recombination site is present between the negative selectable marker gene and the transgene expression cassette, and
- the upstream deletion recombination site is present on the targeting vector between the donor recombination site and the positive selectable marker gene or
- the upstream deletion recombination site is present on the ACE, upstream from the recombination acceptor site and preferably upstream from the promoter.

Preferably, the integrase is a lambda integrase, preferably an ACE integrase and the integrase sites are an AttP-AttB integrase site pair, wherein preferably multiple AttP sites are present on the ACE as recombination acceptor sites wherein the AttB site is present on the loading vector as a recombination donor site.

In a preferred embodiment the gene of interest is a therapeutic gene of interest.

The invention also relates to a method for loading multiple (preferably 3 to 200) genes of interest into an ACE, said method comprising the steps of
i) providing an ACE in a mammalian cell, said ACE having
   - multiple recombination acceptor sites (preferably 3 to 200, more preferably 50-200), preferably integration sites and
   - a promoter directly upstream from the integration site,
ii) obtaining a targeting vector (superloading vector) as defined herein, said targeting vector comprising a gene of interest as defined in any of the above claims,
iii) introducing the targeting vector into said mammalian cell,
iv) integrating said targeting vector into the ACE by providing or using an integrating recombinase (preferably ACE integrase),
v) selecting for the integration event by the positive selectable marker gene,
vi) removing the removable DNA segment between the upstream and downstream deleting recombination sites by providing or using a deletion recombinase in said mammalian cells,
vii) selecting for the removal event by the negative selectable marker gene,
viii) repeating steps ii) to vii) multiple (preferably 1-200) times,
whereby a second or further gene of interest can be loaded by a targeting vector (through the acceptor recombination site(s)) onto said ACE having already one or more integrated genes of interest whereas each integrated genes of interest can be maintained on said ACE.

Preferably in the method of the invention recombination sites are used which are sites as defined herein.

In a preferred embodiment one or both of the recombinases are transiently expressed from an additional expression vector operable in the cell or form the ACE.

The invention also relates to a use of a targeting vector as defined herein for loading (inserting or integrating) multiple genes of interest into an ACE, said ACE having
- multiple recombination acceptor sites (preferably 3-200, more preferably 50-200), preferably integration sites and
- a promoter upstream from the integration site.

The targeting vector is preferably used in a method as defined above.

The invention also relates to a kit for loading genes of interest into an artificial chromosome expression system (ACE) that has been engineered to include multiple sites for site-specific recombination directed integration, said kit comprising at least
- a targeting vector according to the invention, and
- optionally an ACE as defined herein, and/or
- optionally a selecting agent for selection by the positive marker gene, and/or
- optionally a selecting agent for selection by the negative marker gene, and/or
- optionally means for inserting transgenes into the targeting vector, wherein preferably the targeting vector is an empty targeting vector with multicloning site for the cloning of gene of interest. Preferably, said kit further comprises an element selected from the group of:
- the ACE carrying cell line, preferably a mammalian cell line,
- empty superloading vector, i.e. a superloading vector without a transgene
- an integrating recombinase, preferably an integrase or an expression vector for expression thereof, said vector being operable in said mammalian cell,
- a deletion recombinase, preferably a Cre recombinase, or an expression vector for expression said recombinase said vector being operable in said mammalian cell.

The invention also relates to an artificial chromosome expression system (ACE) having multiple (preferably 3 to 200) genes of interest loaded/inserted into pre-engineered sites for site-specific, recombination directed integration.

In a preferred embodiment said ACE can be or is obtained by a method according to the invention.

Preferably, said ACE comprises or carries one or more of the genes of interest without any selectable marker gene being operably linked thereto.

Preferably, in the loaded ACE of the invention at least one gene of interest without any selectable marker gene is present in an expression cassette, said expression cassette comprising at least
- a gene of interest between two integration sites formed upon integration of a targeting vector (superloading vector) as defined in any of claims 1 to 6 said vector carrying the gene of interest,
- a promoter directly upstream from the upstream integration site.

### DEFINITIONS

As used herein a "chromosome" is a nucleic acid molecule with associated proteins, that comprises a centromeric region, telomers and is capable of replication and segregation in a cell upon cell division. Typically natural or endogenous chromosomes (i.e. chromosomes essentially identical to intact chromosomes found in nature or in a non-transformed cell) have a centromeric region, telomeric regions, region(s) of nucleic acids between the centromeric region and the telomeric regions, and origins of replication.

A centromere is a nucleic acid segment with associated proteins, within the chromosome having a nucleic acid sequence that confers an ability on the chromosome to segregate to daughter cells through mitotic and meiotic division. The centromere is the region within the chromosome that provides the foundation for the assembly of kinetochore, a protein structure where upon mitosis or meiosis the spindle fibers attach before segregation of sister chromatids. Typically the DNA sequence of the centromere and the associated proteins together define this function. A given centromere may not necessarily derived from the same species in which it is introduced but should have the ability to ensure segregation in that species. Thereby, this ability of the centromere defines the species specificity of the chromosome. Thus, e.g. a mammalian chromosome, like a mammalian artificial chromosome can replicate and segregate in mammals and also a human chromosome, like a human artificial chromosome can replicate and segregate in humans and mammals.

"Satellite DNA" is a type of DNA sequence which is highly repetitive. Satellite DNA is usually located in regions surrounding the centromere. In the repeat elements of the satellite DNA, the nucleotide sequence may be identical or similar, i.e. largely homologous, for example having an at least 50%, 60%, 70%, 80% 90% or 95%s sequence identity. In humans, the primary centromeric repeat unit is called α-satellite (or alphoid), although a number of other sequence types are found in this region.

The centromere and the satellite DNA optionally forming part of the centromere, are typically associated with proteins forming together heterochromatin, in which the region of satellite DNA is called pericentric or pericentromeric heterochromatin, which is a kind of constitutive heterochromatin, i.e. constitutively packed into a highly condensed form of chromatin and genetically inactive.

A "vector" as used herein is a nucleic acid molecule, preferably DNA (DNA vector), with or without one or more associated protein, carrying or useful for carrying a foreign or pre-defined DNA fragment. Thus the vector comprises means, e.g. a sequence suitable for inserting such a fragment, e.g. a cloning site, e.g. a multicloning site and/or a recombination site. Vectors also comprise an origin of replication and preferably a selectable marker gene. Thus, a vector is useful as a gene delivery vehicle i.e. as a vehicle to transfer genetic material into a cell.

An "artificial chromosome" is a chromosomal vector, which is a chromosome that does not occur in nature in its engineered form, is made by human intervention, and still capable of replicating and segregating alongside natural or endogenous chromosomes upon cell division. Artificial chromosomes are preferably "stable", i.e. they are "stably maintained" in a cell, i.e. at least about 80% or 85%, preferably 90%, more preferably 95% of the cells retain the chromosome upon cell division.

"Amplification-based artificial chromosomes" are artificial chromosomes derived from natural or endogenous artificial chromosomes by "amplification", understood herein as a process in which one or more segments of DNA are duplicated or multiplied to yield multiple copies of similar or identical DNA segments on the same chromosome, preferably joined as tandem or inverted repeats.

"Synthesized artificial chromosomes" are artificial chromosomes that are produced by joining the essential components (at least the centromere, telomeres and origins of replication) in vitro. "Reporter artificial chromosomes" comprise one or more reporter constructs comprising a reporter gene operably linked with a regulatory region.

An "artificial chromosome expression system" (ACes or ACEs) as used herein refers to an artificial chromosome comprising many copies of a recombination site, preferably an integration site for introduction of a DNA segment e.g. an expression cassette comprising a gene of interest and one or more marker genes of which at least one is a selectable marker gene.

A "platform artificial chromosome (expression system)" or "artificial chromosome (expression system) platform" (platform ACEs or ACEs platform) as used herein is an artificial chromosome expression system that has been engineered to include or comprise more than one sites for site-specific, recombination-directed integration.

Preferred artificial chromosomes according to the invention are "satellite DNA based artificial chromosomes" (SATAC), artificial chromosomes which consist essentially of neutral, i.e. genetically inactive and/or non coding and/or not protein coding sequences, i.e. form heterochromatin (or their majority is heterochromatin or they consist essentially of heterochromatin), except engineered or foreign or heterologous sequence segments which preferably comprise one or more genes or one or more sites for insertion of genes.

A "recognition sequence" as used herein is a sequence of nucleotides in a nucleic acid that a nucleic acid binding molecule or complex (a protein, DNA or RNA molecule, or combinations thereof, e.g. restriction endonuclease, modification methylase or a recombinase) recognizes and binds.

"Recombination" is the breaking and rejoining of one or more nucleic acid molecules, preferably DNA strands to form new nucleic acid molecules encoding a novel, preferably rearranged set of genetic information.

A "recombination site" is a definite site in a nucleic acid comprising a recognition sequence where upon binding of said nucleic acid binding molecule or complex, preferably a recombinase, a rearrangement in the nucleic acid occurs. This rearrangement, i.e. the recombination event can be e.g. addition of a nucleic acid segment, i.e. insertion or integration or deletion of a nucleic acid segment or other rearrangement of the nucleic acid sequence. "Integration" is a type of recombination (event) wherein at least two nucleic acid molecules are broken at specific sites and a first (donor) nucleic acid having broken, i.e. partial specific sites at each end is integrated into the broken i.e. partial specific sites of a second (acceptor) nucleic acid thereby forming new nucleic acid molecule comprising the sequence or a part thereof of the first nucleic acid within the sequence or a part thereof of the second nucleic acid. "Removal", "deletion" or "excision" (used interchangeably herein) of a nucleic acid segment from a nucleic acid is a type of recombination (event) wherein said nucleic acid is broken at two specific sites, the segment between the two broken, therefore partial, specific sites is cut and removed and the two partial specific sites are linked together to form a single specific site.

Correspondingly the recombination site can be e.g. an insertion or integration site or a deletion (recombination) site.

The recombination site is unidirectional if recombination can not be reversed, i.e. if said site facilitates e.g. deletion while prevents reintegration of the deleted sequence or facilitates integration while prevents excision of already integrated sequences upon further exposure to the recombinase. As used herein a recombinase is an enzyme that catalyses the exchange of DNA segments, e.g. their removal or deletion, insertion or integration, and/or rearrangement at specific recombination sites. An "integrase" is a recombinase catalysing an integration recombination event. An "integration site" is a donor or an acceptor recombination site of an integrase enzyme.

An "acceptor recombination site" is the specific site of the second (acceptor) nucleic acid into which the first (donor) nucleic acid is integrated.

A "donor recombination site" is the specific site of the first (donor) nucleic acid which is recombined with the acceptor recombination site in the second (acceptor) nucleic acid when broken to arrive at integration of the said first (donor) nucleic acid or a part thereof into the second (acceptor) nucleic acid.

Recombination may require (further) recombination proteins, e.g. excisive protein(s), integrative protein(s), enzymes, associated proteins and co-factor involved in the recombination reaction.

A "positive selectable marker" is a gene that confers selective advantage to the organism in which it is expressed, preferably when a specific condition is provided to said organism (selection), e.g. said organism is contacted with a specific substance.

A "negative selectable marker" is a gene that confers selective disadvantage to the organism in which it is expressed, preferably when a specific condition is provided to said organism (selection), e.g. said organism is contacted with a specific substance. Preferably, the negative selectable marker gene, when expressed, would eliminate the organism upon selection whereas the same organism would survive under selection if the marker gene is not expressed. An example is the thymidine kinase gene, which makes the host sensitive to ganciclovir selection.

### BRIEF DESCRIPTION OF THE FIGURES

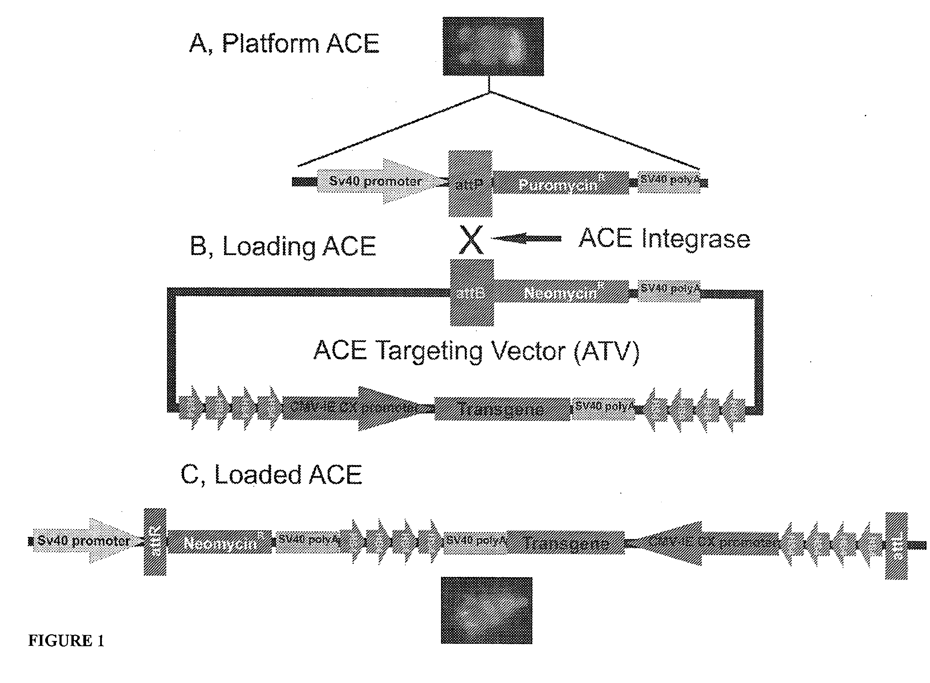

**Figure 1****.** The schematics show the site-specific integration process by which the ACE chromosome was targeted with therapeutic transgenes. (A) The Platform ACE contains multiple attP recombination acceptor sites for the ACE integrase. The attP site is situated between an SV40 promoter and the puromycin resistance gene, which is driven by this promoter. (B) The ATV vectors carry the attB recombination site for the ACE integrase. Immediately after the attB site, a promoterless neomycin resistance gene is cloned. The ACE integrase catalyzes the site-specific recombination between the attB and attP sites and integrates the ATV into the tAC. (C) This event disconnects the puromycin resistance gene from its promoter and replaces it with the promoterless neomycin resistance gene, which in this way acquires the SV40 promoter. Targeted, transgene-carrying cell lines can be selected for neomycin resistance.

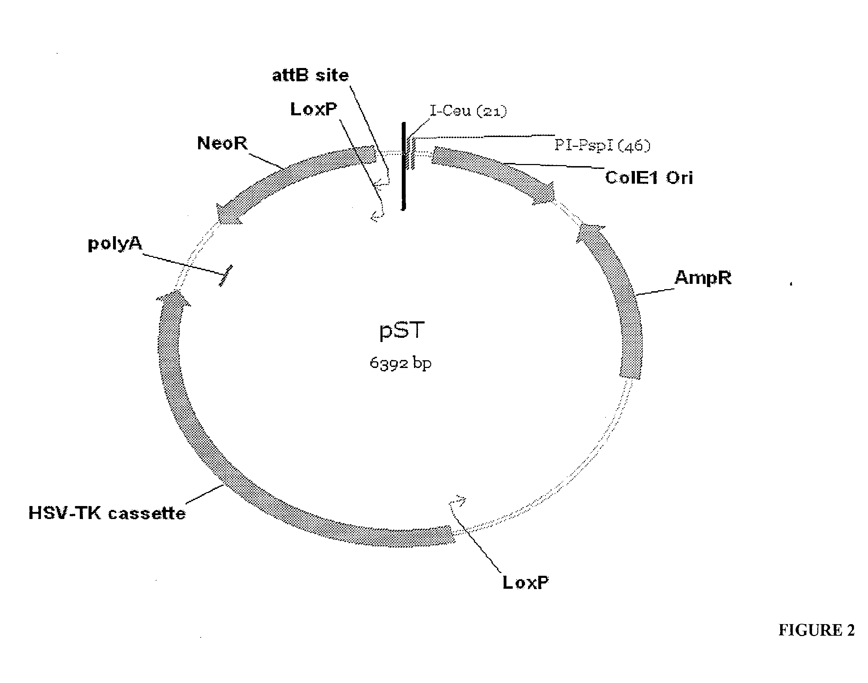

**Figure 2****.** The pST plasmid vector was produced to achieve "superloading" of Platform-ACE chromosomes. This plasmid contains a promoterless neomycin gene and a thymidine kinase expression cassette flanked by direct repeats of two loxP sites. After loading a transgene in pST vector onto the Platform-ACE chromosome as it was previously described (Lindenbaum et al., 2004), the loxP flanked selectable marker gene cassette could be removed by a transient enzymatic action of Cre recombinase. Cell lines, lacking the neomycin-thymidine kinase selectable marker gene cassette, could be isolated by using ganciclovir selection.

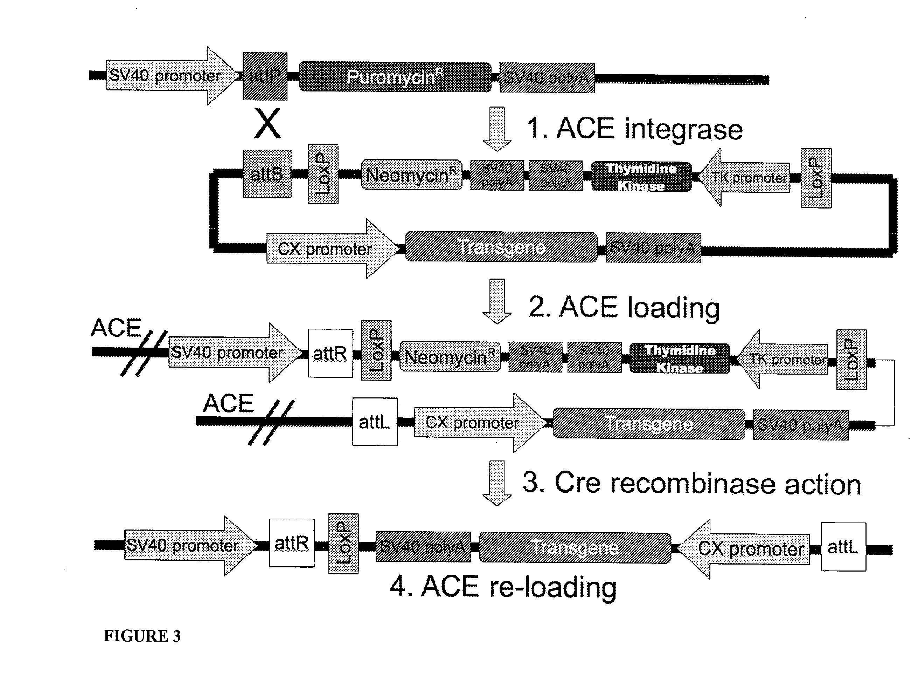

**Figure 3****.** The "superloading" cycle is demonstrated on this cartoon. The transgene is cloned into the pST plasmid and loaded onto the ACE chromosome as shown on Figure 1. In the third step Cre recombinase enzyme is transiently expressed in the cell line carrying the transgene loaded ACE. The Cre recombinase removes the neomycin-thymidine kinase selectable marker gene cassette that is found between the direct repeats of loxP sites. Transgene loaded ACE chromosome carrying cell lines that are lacking the neomycin-thymidine kinase selectable marker gene cassette could be obtained by ganciclovir selection. The ACE chromosome is suitable now for the loading of a new transgene that is cloned into the pST plasmid vector.

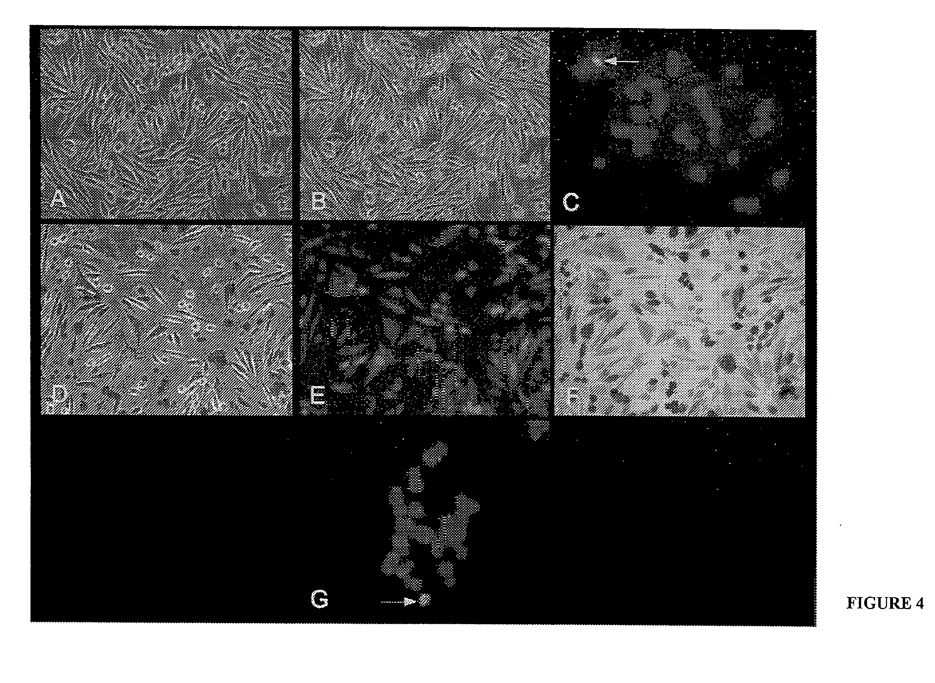

**Figure 4****.** The figure shows the experimental demonstration of the "superloading" process. **A,** 1D9-16 cells are shown on a phase-contrast microscopy picture (Magnification(M): 200x). **B,** 1D9-16 cells emit red fluorescence that is coming from the mCherry protein expressed from the loaded ACE chromosome. **C,** 1D9-16 cells carry the mCherry loaded ACE chromosome that is demonstrated by FISH experiments with a FITC labeled plasmid probe (green signal, yellow arrow). All chromosomes are counterstained with DAPI (blue signal)(M: 630x). **D,** RFPG18-12 cells are shown on a phase-contrast microscopy picture (Magnification(M): 200x). **E,** RFPG18-12 cells emit red fluorescence that is coming from the mCherry protein expressed from the loaded ACE chromosome (M: 200x). **F,** RFPG18-12 cells are stained blue by LacZ staining procedure. This data demonstrates that the second loading with beta-galactosidase was successful and the beta-galactosidase enzyme is expressed from the "superloaded" ACE chromosome and functional (M: 200x). **G,** RFPG18-12 cells carry the mCherry and beta-galactosidase loaded ACE chromosome that is demonstrated by FISH experiments with a FITC labeled plasmid probe (green signal, yellow arrow). All chromosomes are counterstained with DAPI (blue signal)(M: 630x).

### DETAILED DESCRIPTION OF THE INVENTION

Artificial chromosomes are attractive tools for the better understanding of chromosome structure and function. Moreover, artificial chromosomes have a potential use as gene delivery vectors in various fields of gene technology, e.g. gene therapy. In the last decade, a methodology for the in vivo generation of so called mammalian satellite DNA based artificial chromosomes (SATACs) with defined genetic content has been developed. This technology is based on the finding that *de novo* centromere formation can be initiated by targeted integration of any exogenous DNA sequence into the satellite/rDNA region of certain host chromosomes of mammalian cells and thereby self-replicating artificial chromosomes can be obtained. The *de novo* formed new chromosomes comprise exogenous DNA as well as endogenous satellite DNA and also rDNA sequences.

It was found that these mammalian artificial chromosomes, preferably the SATACs, provide a suitable chromosomal environment for stable, persisting expression of the integrated exogenous genetic material. SATACs represent a novel protein production platform both for cellular protein production and for production of e.g. therapeutic molecules in transgenic animals.

Previously, so-called platform ACE chromosomes with multiple recombination recognition sites for a special lambda integrase (ACE integrase) were constructed [Lindenbaum M. et al. Nucleic Acids Research, 32(21) e172 (2004)]. A vector system was also established to deliver "useful genes" onto this Platform ACE. An example of this system is shown on Figure 1. The system includes an entry vector that contains an expression cassette including a multiple cloning site for inserting a gene of interest (Figure 1). This expression cassette is moved then into the ACE targeting vector (ATV) by using yeast homing endonucleases (Figure 1). The ATV plasmid also carries a selectable marker gene (in this example neomycin) without a promoter. This vector has the recombination recognition site for the ACE integrase just upstream from the translation start of the selectable marker gene (Figure 1). The ATV vector harboring the gene of interest is then co-transfected with a plasmid that expresses ACE integrase into a Platform-ACE carrying cell line. The ACE integrase catalyzes recombination between its recognition site on the Platform-ACE and on the ATV vector. This recombination is working in one direction (i.e. unidirectional), i.e. the ATV vector integrates into the ACE chromosome by site specific recombination event. This event results in the so called "loaded" ACE chromosome, where the gene of interest is built into the ACE (Figure 1). It is possible to select for this event, because the site specific recombination is cleverly designed so that the promoterless neomycin marker gene gets a promoter and thereby the "loaded" ACE chromosome containing cells lines could be selected by Geneticin resistance.

While the Platform-ACE chromosome has about 50 to 200 loading sites, which is alone very impressive, however, delivery vectors of this kind suffer from certain limitations. Specifically, these vectors can be loaded with as many gene of interest as many selectable marker genes are available and that number is seriously limited. This limits the number of useful genes that can be loaded onto the artificial chromosome. However, loading multiple genes onto the ACE chromosome may well be important (e.g. in multiple transgenesis experiments, examinations and understanding of biochemical pathways, development of therapeutic applications for cancer and complex diseases). There is a limited number of selection marker genes available and also there is a risk of serious side effects caused by the unwanted expression of these marker genes in mammalian cells, organs and organisms. Furthermore, some of the selectable marker genes may have unwanted side effects when they are expressed in high copy number in certain tissues and organs in the body. These facts limit the therapeutic applications of the ACE system.

The present inventors have invented a novel method that makes it possible to load unlimited number of genes onto ACEs by using only two selectable marker genes that could be removed from the ACEs, before therapeutic applications.

Thus, a new vector system is disclosed herein which can utilize only one selectable marker gene cassette for the delivery of the gene of interest and this vector can be recycled and used for new gene loading by utilizing a simple method. The genes of interest can be loaded onto mammalian artificial chromosomes, preferably an artificial chromosome expression system (ACEs), which comprise unidirectional recombination sites, preferably integrase recognition sites and promoters directly upstream from the recombination sites. It is possible to select for this event, because the site specific recombination is designed so that the positive selectable marker is promoterless in the selectable marker gene cassette of the targeting vector (the loading vector or "superloading" vector) whereas gets a promoter upon integration into the chromosome and thereby the "loaded" ACE chromosome containing cells lines can be selected by the positive selectable marker. The promoterless positive selectable marker is therefore located just downstream from the integrase recognition site, i.e. sufficiently close to the promoter after integration so that expression may occur, i.e. the promoter and the gene become operably linked.

The targeting vector also comprises a negative selectable marker gene together with an appropriate promoter and optionally other sequences facilitating expression of this marker gene. The negative selectable marker gene is transcribed from the opposite DNA strand compared to the positive selectable marker gene.

The two selectable marker genes are flanked by two recombination sites which are or capable of functioning as deletion recombination sites. To the contrary, the expression cassette of the gene of interest is not within the removable DNA segment i.e. between the deletion recombination sites.

In an embodiment both deletion recombination sites can be found on the targeting (superloading) vector.

So that integration may occur the presence of an appropriate integrase corresponding to the integration recombination sites is to be ensured. When integration recombination occurred the positive selectable marker is expressed and the loaded artificial chromosomes can be effectively selected. After selection of the correct constructs the removable DNA segment comprising the two selectable markers is to be removed. To that end the presence of a deleting recombinase effective on the deletion recombination sites is to be ensured. After the removable DNA segment removed both selectable marker has been removed from the artificial chromosome whereas the transgene, i.e. the gene of interest remains loaded to the chromosome. The success of the deletion recombination event can be effectively controlled by the negative selectable marker, which selects for the lack of the presence of the expression of this marker gene.

In a further round of loading the same type of targeting vector with the same selectable markers can be applied and either the same or a different gene of interest can be loaded onto one or more further insertion recombination site(s) of the artificial chromosome.

As a platform-ACE chromosome (platform ACEs) has about 50 to 200 loading sites, a large number of genes can be loaded onto this vector with the use of only two selectable marker genes. Moreover, no unnecessary foreign sequences remain on the loaded artificial chromosome, which provides a further advantage, in particular in gene therapy applications.

Thus, the elements of the vector system of the invention are
- an artificial chromosome expression system, preferably a platform artificial chromosome expression system comprising at least one preferably a multiplicity of recombination integration sites, and having a promoter just upstream from the recombination site(s) in a position suitable to drive expression of a positive selectable marker gene downstream from the integration site once integration occurs, and
- a targeting vector called here a superloading vector, comprising a promoterless positive selectable marker gene just downstream from the recombination site and, on the opposite strand and arranged in the opposite direction a negative selectable marker gene expression cassette, and a transgene expression cassette comprising or suitable for insertion of a transgene (gene of interest), the selectable marker genes being flanked by a pair of deletion recombination sites thereby forming a removable DNA segment.

Optionally the system comprises further elements as follows.
- Expression vector(s) to provide expression a recombinase for integration of the targeting vector (integrase) in the cell having the artificial chromosome.
- Expression vector(s) to provide expression of a recombinase for removal of the removable gene segment.
- Selective agent for the negative selectable marker.
- Selective agent for the positive selectable marker.
- If the superloading vector, as used or marketed comprises a site for insertion of the transgene, e.g. a multi-cloning site but does not comprise the transgene itself, means for insertion of the transgene or one or more transgenes of interest.

The recombinases (integrases and/or deletion or excision recombinases) useful in the present invention can be provided by expression from expression vectors. Alternatively, the cells carrying the artificial chromosome may be engineered to express said recombinases.

### Recombination sites

A variety of recombination sites can be applied according to the present invention.

As an integration recombination site for example, the att site can be applied as described among other in US2012064578 or in Lindenbaum M. et al. Nucleic Acids Research, 32(21) (2004). In a preferred embodiment of the invention attB and attP sites are applied which, upon the unidirectional recombination, i.e. integration are converted onto *attR* and *attL* sites. The preferred integrase catalyzing integration is a bacterial lambda phage integrase specific to these sites. However, other integrases as integrases from R4, TP901-1 and Bxb1 phages were found to mediate DNA recombination to attB and attP sites in mammalian cells [Yamaguchi S et al. PLoS ONE 6(2) 17267 (2011)]. The recombination site attB is an approximately 33 base pair sequence containing two 9 base pair core type integrase binding sites and a 7 base pair long overlap region, whereas the attP site is approximately 240 base pair long and comprises sites for the integrase as well as sites for auxiliary proteins [see Landy A. Curr Opin Genet Dev. 3(5) 699-707. (1993)].

Further integration recombination systems may be applicable in the present invention, as well [as an example see Nern, A et al. PNAS 108(34) 14198-14203 (2011).

A deletion recombination site is a recombination site, which is suitable for deletion of a pre-selected DNA segment flanked by the recombination sites or specific elements thereof.

A deleting recombination site can be a bi-directional recombination site if applied so that a DNA segment flanked by the recombination sites or site elements can be removed from a DNA molecule comprising said segment.

An example for deleting recombination site is the loxP sites of the Cre/lox site-specific recombination system provided that two lox sites flanking the removable DNA segment are present in the same orientation on the DNA molecule.

The Cre/lox recombination system is a system capable of inducing the following three types of recombination events:
a) deletion of a pre-selected DNA segment flanked by lox sites in the same orientation,
b) inversion of a pre-selected DNA segment flanked by lox sites in the opposite orientation,
c) reciprocal exchange of DNA segments proximate to lox sites located on different DNA molecules.

According to a preferred embodiment of the present invention the Cre/lox system is applied as an unidirectional deleting recombination system, i.e. the lox sites (preferably two lox sites) are present only on the superloading vector (i.e. *in cis)* in the same orientation flanking the DNA segment to be removed so as to avoid both inversion and reciprocal exchange.

A description and review of the application of this system can be found e.g. in Nagy Andras "Cre Recombinase: The Universal Reagent for Genome Tailoring" genesis 26:99-109 (2000).

Other recombination systems may be useful in removal of the DNA segment according to the invention. An other system applicable in the present invention is the FLP/frt recombination system described e.g. in US2008295192A1 [Thomas K. R. et al., (2008)].

### Artificial chromosomes

In the present invention in principle any artificial chromosome expression system (ACEs) as defined herein can be applied provided that it meets the requirements as described herein for integration of the targenting vector or a part.

Preferred ACEs are those disclosed by Lindenbaum et al. in US2012064578.

As a basis for artificial chromosome, nevertheless, a number of types of artificial chromosomes can be applied e.g. those described in patent publications US5712134, US5891691, US5288625, US6743967, for example minichromosomes or megachromosomes.

Highly preferred artificial chromosomes according to the invention are mammalian artificial chromosomes.

Preferred basis for ACEs of the invention are SATACs that can be engineered in different mammalian species including humans, and can be purified and transferred into many types of recipient somatic cells and zygotes. Transgenic animals have successfully been generated with purified SATACs, and the transmission of the artificial chromosome through several generations has been achieved. Moreover, tissue-specific expression of a therapeutic gene in transgenic offspring has been demonstrated.

Stable and heritable SATACs with large carrying capacity may serve as potential vectors for animal breeding and in production of humanized cells, tissues and organs for xenotransplantation.

*In vitro* synthesized artificial chromosomes are artificial chromosomes that are produced by joining the essential components (at least the centromere, and origins of *replication) in vitro.*

Preferred engineered chromosomes are amplification-based artificial chromosomes obtained by a so-called amplification event induced by introduction of heterologous nucleic acid into ribosomal DNA (rDNA) in a chromosome, resulting in chromosomal fragments comprising repeated segments, i.e. an ordered segmentation that can be visualized by known techniques, e.g. by anti-centromere antibodies and which cannot be observed in endogenous chromosomes.

### Selectable markers

In the present specification exemplary selectable markers are e.g. the neomycin and thymidine kinase selectable gene cassettes, which are preferred.

Further positive and negative selectable markers are well known for a person skilled in the art and are disclosed in many publications, like Vile R. "Selectable Markers for Eukaryotic Cells" Practical Molecular Virology: Viral Vectors for Gene Expression Series: Methods in Molecular Biology 8 (1991) 49-60 and in Mortensen, R., Chestnut, J. D., Hoeffler, J. P. and Kingston, R. E. 2001. Selection of Transfected Mammalian Cells. Current Protocols in Neuroscience. 4.6.1-4.6.20.

The invention and its certain embodiments are further illustrated below by preferred, non-limiting examples.

### EXAMPLES

### EXAMPLE 1 - Materials and methods

### Plasmids

The pSTRFP plasmid was produced by cloning the coding sequence of mCherry gene into the pST plasmid vector (from pmR-mCherry, Clontech, 632542). The pSTLZ plasmid was produced by cloning the coding sequence of beta-galactosidase gene into the pST plasmid vector (from pCH110, Amersham). The pCre-GFP plasmid was used to transiently express Cre recombinase to remove the neomycin-thymidine kinase selectable marker gene cassette flanked by direct loxP sites from ACE chromosomes (gift from Jim Downing's laboratory and available from Clontech Laboratories, Inc.). The pCXLamIntROK plasmid vector was used to transiently express ACE integrase for site-specific loading transgenes onto ACE chromosomes as previously described Lindenbaum M. et al. Nucleic Acids Research, 32(21) (2004)

### Cell culture

The Platform ACE-carrying Y2913D-SFS Chinese hamster ovary (CHO DG44) cell line was obtained from Chromos Molecular Systems Inc. It was cultured in MEM alpha (Gibco, 22571), 5% FCS, streptomycin-penicillin (Gibco, 15070 - 063) and 10 mg/ml Puromycin (Sigma, P-7255). 1D9-16, RFPG18 and RFPG18-12 cell lines were cultured in the same medium as described above except that Puromycin was omitted. 1D9-16 and RFPG18-12 cell lines were cultured in the presence of 400 ug/ml G418 (Sigma, G-5013). RFPG18 cell line was cultured in the presence of 10 um ganciclovir (Sigma, G-2536).

### Cell transfection

Plasmid transfection experiments were performed with the Superfect reagent (Qiagen, 301305) as described by the manufacturer.

### FISH

Fluorescence in situ hybridization (FISH) experiments were performed with a standard protocol. The following DNA sequence was labeled with FITC fluorescent dye and used as a probe: pPur (Clontech, 631601) plasmid for ACE detection. DNA probes were labeled either with Roche DIG Nick Translation Mix (1745816) or with Roche Biotin Nick Translation Mix (1745824).

### PCR

Genomic DNA samples were isolated with the Wizard Genomic DNA Purification Kit (Promega, A1125). Site-specific integration of transgenes into the ACE chromosome was detected by PCR experiments with the following primers: 193AF: 5'-ACCCCCTTGCGCTAATGCTCTGTTA and NeoRl 5'-TCGATGAATCCAGAAAAGCGGCCA. The expected product size is 818 bp.

### Microscopy

All pictures were photographed under a Zeiss Axiovision Z1 fluorescent microscope by using the Axiovision software that was purchased with the microscope.

### EXAMPLE 2 - Results and discussion

In this example, we report the development of a new vector system which is using only one selectable marker gene cassette for the delivery of the gene of interest and this vector can be recycled and used for new gene loading by utilizing a simple method. The new selectable marker gene cassette consists of a promoterless neomycin gene just downstream from the integrase recognition site and a thymidine kinase (derived from *Herpes simplex* virus) expression cassette including an SV40 promoter and an SV40 polyadenylation signal. The thymidine kinase expression unit is transcribed from the opposite DNA strand compared to the neomycin gene. The neomycin and thymidine kinase selectable gene cassette is flanked by loxP sites in direct orientation. LoxP is the recognition site for the site specific recombination enzyme, Cre. Cre enzyme is able to catalyze recombination between these two loxP sites, and by this event the whole selectable marker gene cassette is removed. This phenomenon is exploited in our gene loading system. This new type of ATV plasmid is called "superloading" vector (pST, Figure 2) and on overview of the "superloading" cycle is presented on Figure 3.

The first gene of interest is cloned into the superloading vector through the steps described before⁷. Since we report here a proof of concept experiment, we have chosen the coding sequence of mCherry red fluorescent protein *(Discosoma sp*.) as the first gene to load onto ACE and constructed the pSTRFP superloading vector. Then this superloading vector was transfected into the ACE carrying cell line along with the ACE integrase expressing vector. Twenty-four hours after transfection antibiotic selection was started by Geneticin to grow colonies that are carrying the superloading vector correctly targeted onto the ACE. Correct targeting to ACE was verified by PCR reactions specific to site-specific integration on purified genomic DNA samples from Geneticin antibiotic resistant cell lines. Cell lines were also examined for the presence of red fluorescence coming from mCherry protein through fluorescent microscopy (Figure 4B). The ACE chromosomes were also examined for the presence of the gene of interest, size and chromosomal intactness by FISH analysis (Figure 4C). On the basis of correct ACE targeting, a good fluorescent signal and a good sized, intact ACE chromosome, we have chosen a single cell line (1D9-16) for further experiments. Ganciclovir is a synthetic analogue of 2'-deoxy-guanosine. It is first phosphorylated to a deoxyguanosine triphosphate (dGTP) analogue by the viral thymidine kinase we have delivered onto ACE by the pSTRFP vector. This competitively inhibits the incorporation of dGTP by DNA polymerase, resulting in the termination of elongation of DNA replication, which eventually results in cell death. We have determined the minimum effective concentration of ganciclovir that was sufficient to kill 1D9-16 cells and found that it was 1 uM. A Cre recombinase expressing plasmid was transfected into the 1D9-16 cells and Cre recombinase was transiently expressed. Since the neomycin and thymidine kinase expression cassette is flanked by loxP sites in direct orientation, we expected that Cre recombinase would remove this marker gene construct from the ACE chromosome effectively leaving behind our gene of interest only, which in this case is mCherry. Twenty-four hours after transfection ganciclovir selection was started at 1 uM concentration and resistant cell lines were isolated. Red fluorescence emitting cell lines were further examined by FISH experiments for the presence of an intact ACE chromosome as previously described. A cell line, RFPG18, was chosen for further experiments. We have constructed a new superloading vector into which we have inserted the coding sequence for the bacterial beta-galactosidase gene (LacZ). This new DNA construct was called pSTLZ and it had contained the same selectable marker gene cassette flanked by direct repeats of loxP sites as the pSTRFP vector. The pSTLZ plasmid was co-transfected with the ACE integrase expressing plasmid into the RFPG18 cell line. Geneticin resistant cell lines were isolated. Correct ACE targeting was determined by site-specific PCR experiments. Cell lines were also tested for the positivity of LacZ staining (Figure 4F). Next we have sorted out those cell lines that demonstrated correct ACE targeting, were positive for LacZ staining (Figure 4F) and also emitted red fluorescence (Figure 4E). These cell lines were further analyzed for the size and chromosomal intactness of ACE chromosomes by FISH analysis (Figure 4G).

We have identified cell lines with intact ACE chromosomes carrying and functionally expressing both mCherry and LacZ genes correctly targeted onto the artificial chromosome alone. In these experiments we have used only one selectable marker gene cassette to deliver two different useful genes onto the ACE chromosome. The procedure presented here is easily fulfilled, highly efficient, requires minimal labour and present no serious toxicity to cells. Also, there is the possibility to remove all the selectable marker gene copies that were used to deliver useful genes onto the ACE at the last step before therapeutic applications. This reduces the risk of side effects coming from the expression of genes, which are coding for antibiotic resistance in the body of a possible future patient.

### INDUSTRIAL APPLICABILITY

The novel method and system according to the invention makes it possible to load a practically unlimited number of genes onto ACEs by using only two selectable marker genes that could be removed from the ACEs.

The invention is useful in efficient production of gene products including pharmaceutical proteins, transgenic plants and animals.

Gene therapy might be an extremely promising treatment offered for a vast amount of genetic and acquired disorders in the near future. Presently, there is still a significant hindrance in the clinical success of this revolutionary medicine: the lack of a safe, stable and reliable genetic vector.

This novel technology could revolutionize gene therapeutic applications aimed at the treatment of complex disorders and cancers. It could also speed up cell therapy by allowing researchers to engineer a chromosome with a predetermined set of genetic factors that could help differentiate adult stem cells, embryonic stem cells and iPS cells into adult cell types with therapeutic value.

### REFERENCES

| |
|---|
| Seymour, L. W. & Fisher, K.D. Pr-eclinical Screening of Gene Therapy in Human Tissues. Human Gene Therapy 20, 291-292 (2009*).* |
| Hadlaczky, G. et al. Centromere formation in mouse cells cotransformed with human DNA and a dominant marker gene. Proc.Natl.Acad.Sci.U.S.A 88, 8106-8110 (1991). |
| Praznovszky, T. et al. De novo chromosome formation in rodent cells. Proc. Natl. Acad. Sci. U.S.A 88, 11042-11046 (1991). |
| Kereso, J. et al. De novo chromosome formations by large-scale amplification of the centromeric region of mouse chromosomes. Chromosome Res 4, 226-239 (1996*).* |
| Hollo, G. et al. Evidence for a megareplicon covering megabases of centromeric chromosome segments. Chromosome Res 4, 240-247 (1996*).* |
| Csonka, E. et al. Novel generation of human satellite DNA-based artificial chromosomes in mammalian cells. J.Cell Sci 113 (Pt 18), 3207-3216 (2000). |
| Lindenbaum, M. et al. A mammalian artificial chromosome engineering system (ACE System) applicable to biopharmaceutical protein production, transgenesis and gene-based cell therapy. Nucleic Acids Res 32, e172 (2004). |
| Katona, R.L. et al. A combined artificial chromosome-stem cell therapy method in a model experiment aimed at the treatment of Krabbe's disease in the Twitcher mouse. Cell. Mol. Life Sci 65, 3830-3838 (2008). |
| Duncan, A. & Hadlaczky, G. Chromosomal engineering. Curr. Opin. Biotechnol 18, 420-424 (2007). |
| Hadlaczky, G. Satellite DNA-based artificial chromosomes for use in gene therapy. Curr:Opin.Mol.Ther 3, 125-132 (2001). |
| Lindenbaum M. et al. "A mammalian artificial chromosome engineering system (ACE System) applicable to biopharmaceutical protein production, transgenesis and gene-based cell therapy" Nucleic Acids Research, 2004, Vol. 32, No. 21 |
| Kuroiwa Y, Tomizuka K, Shinohara T, Kazuki Y, Yoshida H, Ohguma A, Yamamoto T, Tanaka S, Oshimura M, Ishida I: Manipulation of human minichromosomes to carry greater than megabase-sized chromosome inserts. Nature Biotechnol (2000) 18:1086-1090 |
| Yamaguchi S et al. "A Method for Producing Transgenic Cells Using a Multi-Integrase System on a Human Artificial Chromosome Vector" PLoS ONE 6(2) 17267 (2011) |
| Nagy Andras "Cre Recombinase: The Universal Reagent for Genome Tailoring" genesis 26:99-109 (2000) |
| Nern, A, Pfeiffer B D., Svoboda K, and Rubin G M., "Multiple new site-specific recombinases for use in manipulating animal genomes" PNAS 108(34) 14198-14203 (2011) |
| Kennard M. L. "Engineered Mammalian Chromosomes in Cellular Protein Production: Future Prospects" in Mammalian Chromosome Engineering; Methods in Molecular Biology 738 (2011) 217-238 |
| Landy A. "Mechanistic and structural complexity in the site-specific recombination pathways of Int and FLP" Curr Opin Genet Dev. 3(5) 699-707. (1993) |
| Vile R. "Selectable Markers for Eukaryotic Cells" Practical Molecular Virology: Viral Vectors for Gene Expression Series: Methods in Molecular Biology 8 (1991) 49-60 |
| Mortensen, R., Chestnut, J. D., Hoeffler, J. P. and Kingston, R. E. 2001. Selection of Transfected Mammalian Cells. Current Protocols in Neuroscience. 4.6.1-4.6.20. |
| Patents and patent publications: |
| EP0473253 |
| US2012064578 |
| US6077697 |
| US6025155 |
| WO2002097059 |
| WO1997040183 |

## Claims

1. A system for loading genes of interest into an artificial chromosome expression system (ACE) that has been engineered to include multiple sites for site-specific, recombination directed integration said system comprising at least
i) an ACE having
- multiple (preferably 2 to 200, more preferably 50 to 200) recombination acceptor sites and
- a promoter directly (preferably at most 43 bp) upstream from the recombination acceptor sites, and
ii) a targeting vector (superloading vector), said targeting vector having
- a recombination donor site,
- a transgene expression cassette for the expression of a gene of interest comprising at least one gene of interest or at least one cloning site for a gene of interest operably linked with a promoter-enhancer element allowing expression of the gene(s) of interest
- a positive selectable marker gene directly downstream from the recombination donor site so that said positive selectable marker gene is expressed under the control of the promoter upstream from the recombination acceptor site of the ACE in a mammalian cell, once the targeting vector has been integrated into the ACE to form a loaded ACE
- a negative selectable marker gene expression cassette including promoter sequence and poly-A signal to ensure the expression of the negative selectable marker gene,
wherein the loaded ACE comprises a removable DNA segment, said removable DNA segment being
- flanked by an upstream and a downstream deletion recombination site for removal of said DNA segment from the loaded ACE upon the enzymatic action of a deleting recombinase enzyme specific to (capable to fulfill of its enzymatic reaction on) the deleting recombination sites thereby removing the removable DNA segment, and
- comprising both selectable marker genes.

2. A targeting vector for loading genes of interest into an artificial chromosome expression system (ACE) that has been engineered to include multiple sites for site-specific, recombination directed integration, said ACE having
- multiple (preferably 2 to 200, more preferably 50 to 200) recombination acceptor sites and
- a promoter directly (preferably at most 43 bp) upstream from the recombination acceptor sites, and said targeting vector (superloading vector) having
- a recombination donor site,
- a transgene expression cassette for the expression of a gene of interest comprising at least one gene of interest or at least one cloning site for a gene of interest operably linked with a promoter-enhancer element allowing expression of the gene(s) of interest
- a positive selectable marker gene directly downstream from the recombination donor site so that said positive selectable marker gene is expressed under the control of the promoter upstream from the recombination acceptor site of the ACE in a mammalian cell, once the targeting vector has been integrated into the ACE to form a loaded ACE
- a negative selectable marker gene expression cassette including promoter sequence and poly-A signal to ensure the expression of the negative selectable marker gene,
wherein the loaded ACE comprises a removable DNA segment, said removable DNA segment being
- flanked by an upstream and a downstream deletion recombination site for removal of said DNA segment from the loaded ACE upon the enzymatic action of a deleting recombinase enzyme specific to (capable to fulfill of its enzymatic reaction on) the deleting recombination sites thereby removing the removable DNA segment, and
- comprising both selectable marker genes.

3. The system according to claim 1 or the targeting vector according to claim 2 wherein both the upstream and downstream deletion recombination sites are LoxP recombination sites in direct orientation for the enzymatic action of Cre recombinase enzyme, which enzyme if recquired is capable to fulfill of its enzymatic reaction on the LoxP sites and by this step both the positive selectable marker gene and the negative selectable marker gene cassette can be removed from the ACE chromosome.

4. The system according to claim 1 or the targeting vector according to claim 2 wherein
- the downstream deletion recombination site is present between the negative selectable marker gene and the transgene expression cassette, and
- the upstream deletion recombination site is present on the targeting vector between the donor recombination site and the positive selectable marker gene or
- the upstream deletion recombination site is present on the ACE, upstream from the recombination acceptor site and preferably upstream from the promoter.

5. The system according to claim 1 or the targeting vector according to claim 2, wherein the integrase is a lambda integrase, preferably an ACE integrase and the integrase sites are an AttP-AttB integrase site pair, wherein preferably multiple AttP sites are present on the ACE as recombination acceptor sites wherein the AttB site is present on the loading vector as a recombination donor site.

6. The targeting vector as defined in any of the above claims, wherein said gene of interest is a therapeutic gene of interest.

7. A method for loading multiple (preferably 3 to 200) genes of interest into an ACE, said method comprising the steps of
i) providing an ACE in a mammalian cell, said ACE having
- multiple recombination acceptor sites (50-200), preferably integration sites and
- a promoter directly upstream from the integration site,
ii) obtaining a targeting vector (superloading vector) as defined in any of claims 1 to 6, said targeting vector comprising a gene of interest as defined in any of the above claims,
iii) introducing the targeting vector into said mammalian cell,
iv) integrating said targeting vector into the ACE by providing or using an integrating recombinase (preferably ACE integrase),
v) selecting for the integration event by the positive selectable marker gene,
vi) removing the removable DNA segment between the upstream and downstream deleting recombination sites by providing or using a deletion recombinase in said mammalian cells,
vii) selecting for the removal event by the negative selectable marker gene,
viii) repeating steps ii) to vii) multiple (preferably 1-200) times,
whereby a second or further gene of interest can be loaded by a targeting vector (through the acceptor recombination site(s)) onto said ACE having already one or more integrated genes of interest whereas each integrated genes of interest can be maintained on said ACE.

8. The method according to claim 7 wherein the recombination sites are as defined in any of claims 3 to 5.

9. The method according to claim 7 or 8 wherein one or both of the recombinases are transiently expressed from an additional expression vector operable in the cell or form the ACE.

10. Use of a targeting vector as defined in any of claims 1 to 6 for uploading multiple genes of interest into an ACE, said ACE having
- multiple recombination acceptor sites (50-200), preferably integration sites and
- a promoter upstream from the integration site,
preferably in a method as defined in any of claims 7 to 9.

11. A kit for loading genes of interest into an artificial chromosome expression system (ACE) that has been engineered to include multiple sites for site-specific, recombination directed integration, said kit comprising at least
- a targeting vector according to any of claims 2 to 6, and
- optionally an ACE as defined in any of claims 1 to 6, and/or
- optionally a selecting agent for selection by the positive marker gene, and/or
- optionally a selecting agent for selection by the neative marker gene, and/or
- optionally means for inserting transgenes into the targeting vector, wherein preferably the targeting vector is an empty targeting vector with multicloning site for the cloning of gene of interest.

12. The kit according to claims 11 wherein said kit further comprises an element selected from the group of:
- the ACE carrying cell line, preferably a mammalian cell line,
- empty superloading vector
- an integrating recombinase, preferably an integrase or an expression vector for expression thereof, said vector being operable in said mammalian cell,
- a deletion recombinase, preferably a Cre recombinase, or an expression vector for expression said recombinase said vector being operable in said mammalian cell.

13. An artificial chromosome expression system (ACE) having multiple (preferably 3 to 200) genes of interest loaded/inserted into pre-engineered sites for site-specific recombination directed integration, said ACE being obtainable by a method according to any of claims 7 to 9.

14. An ACE having multiple (preferably 3 to 200) genes of interest integrated into pre-engineered sites for site-specific recombination directed integration, wherein one or more of the genes of interest without any selectable marker gene being operably linked thereto.

15. The ACE according to any of claims 13 or 14 wherein the at least one gene of interest without any selectable marker gene is present in an expression cassette, said expression cassette comprising at least
- a gene of interest between two integration sites formed upon integration of a targeting vector (superloading vector) as defined in any of claims 1 to 6 said vector carrying the gene of interest,
- a promoter directly upstream from the upstream integration site.
